# EUROPEAN PATENT APPLICATION

(11) **EP 4 773 145 A1**
(43) Date of publication of application: **08.07.2026**
(21) Application number: 25150327.2
(22) Date of filing: 06.01.2025
(51) Int. Cl.: G16H 30/20, G16H 30/40, G16H 40/63

(54) **A MEDICAL METHOD FOR PROVIDING A TASK MONITOR SYSTEM FOR IMAGE PROCESSING APPLICATIONS**

(71) Applicant: Koninklijke Philips N.V., 5656 AE Eindhoven (NL)
(72) Inventor: ANAND, Shreya, Eindhoven (NL); MYSORE SIDDU, Dinesh, Eindhoven (NL); ADAK, Saptakatha, 5656AG Eindhoven (NL); PRAKASH, Aman, Eindhoven (NL); SELVAM, Chitrai Senthamil, Eindhoven (NL)
(74) Representative: Philips Intellectual Property & Standards

(57) **Abstract**

Disclosed herein is a medical method. The method comprises retrieving (200) application usage data (126) from a log (122). The application usage data (126) is descriptive of user interface actions performed during performing at least one medical image generating protocol with at least one image processing application (124). The method further comprises generating (202) protocol-specific lists (128) descriptive of the at least one medical image generating protocol by reformatting the application usage data (126). The method further comprises constructing (204) a semantically encoded database (130) from the protocol-specific lists (128). The method further comprises providing (206) a task monitor system (134) constructed from a general purpose large language model (132) configured to integrate the semantically encoded database (130) into prompt responses.

## Description

### FIELD OF THE INVENTION

The invention relates to medical imaging, in particular to the monitoring of image processing applications.

### BACKGROUND OF THE INVENTION

In radiology, image processing applications are software tools that enable complex tasks, such as analyzing comex medical images with precision, offering advanced capabilities such as automated segmentation, anomaly detection, image construction, image fusion, image overlaying, computer aided diagnosis, and image enhancement. However, the proliferation of diverse image processing applications, each with its own unique interfaces, functionalities, and learning curves, presents a significant challenge. Radiologists often find it difficult to master multiple software systems while managing their demanding clinical responsibilities.

United States patent publication US 7,673,340 B1 discloses a system and method for monitoring and analyzing user activity of an interactive system, providing insight and recommendations to improve the interactive system based on the user activity. The present invention analyzes user behavior in the context of the structure of the interactive system as experienced by the user, the analysis of business-critical user tasks, the automated generation of recommendations for improving the interactive system, reports on the interactive system's application logic and data, unique visualizations of user behavior through the use of graphical displays, and secure report viewing and creation. The present invention can be applied to number of interactive systems or a combination thereof.

### SUMMARY OF THE INVENTION

The invention provides for a medical method, a computer program product, and a medical system in the independent claims. Embodiments are given in the dependent claims.

In one aspect a medical method is disclosed. The method comprises retrieving application usage data from a log. The application usage data is descriptive of user interface actions performed during performing at least one medical image generating protocol with at least one image processing application. The method further comprises generating protocol-specific lists descriptive of the at least one medical image generating protocol by reformatting the application usage data. The method further comprises constructing a semantically encoded database from the protocol-specific lists. The method further comprises providing a task monitor system constructed from a general purpose large language model configured to integrate the semantically encoded database into prompt responses.

In another aspect, a computer program product is disclosed. The computer program product comprises a computer-readable storage medium having machine-executable instructions embodied therewith. Said machine-executable instructions are configured to implement an example of the medical method.

In another aspect, a medical system is disclosed. The medical system comprises a memory storing machine-executable instructions. The medical system further comprises a computational system. Execution of the machine-executable instructions causes the computational system to retrieve application usage data from a log. The application usage data is descriptive of user interface actions performed during performing at least one medical image generating protocol with at least one image processing application. Execution of the machine-executable instructions further causes the computational system to generate protocol-specific lists descriptive of the at least one medical image generating protocol by reformatting the application usage data. Execution of the machine-executable instructions further causes the computational system to construct a semantically encoded database from the protocol-specific lists. Execution of the machine-executable instructions further causes the computational system to provide a task monitor system constructed from a general purpose large language model configured to integrate the semantically encoded database into prompt responses.

### BRIEF DESCRIPTION OF THE DRAWINGS

In the following preferred embodiments of the invention will be described, by way of example only, and with reference to the drawings in which:
- Fig. 1: illustrates an example of a medical system.
- Fig. 2: shows a flow chart which illustrates a method of using the medical system of Fig. 1.
- Fig. 3: illustrates one use of the medical system of Fig. 1.
- Fig. 4: illustrates one implementation of a task monitor system.
- Fig. 5: illustrates a further example of a medical system.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In one example there is a medical method. The method comprises retrieving application usage data from a log. Application usage data may be a record of the actions performed on a user interface. The log may then be a summary or list of the actions performed using an application. The application usage data is descriptive of user interface actions performed during performing at least one medical image generating protocol with at least one image processing application. A medical image generating protocol may be a selection of steps or processes which are performed and are used to generate a medical image. In the course of generating a medical image one or more different image processing applications may be used to perform the at least one medical image generating protocol. The method further comprises generating protocol-specific lists descriptive of the at least one medical image generating protocol by reformatting the application usage data.

The application usage data may for example be in a machine-readable form or format which is different from a semantically understandable version. The protocol-specific lists may for example be the application usage data converted into a verbal description of the steps performed in order using the at least one image processing application. The method further comprises constructing a semantically encoded database from the protocol-specific lists. This may for example be used to make a database for separate entries which forms a list for each specific protocol. For example, when a user performs a task such as generating a medical image using an image processing application, it may provide a list of actions performed using the user interface to perform that task. After generating the protocol-specific list this specific list of tasks may then be incorporated into the semantically encoded database.

The method further comprises providing a task monitor system constructed from a general purposed large language model configured to integrate the semantically encoded database into prompt responses. Because large language models are so flexible, the prompt responses can be varied and be used for different purposes. One usage, as is described below, is for analyzing or summarizing the data that is contained in the semantically encoded database. Another usage is to provide a guide to a user and/or monitor the "clicks" or usage of a user interface when an operator is guided or instructed on how to construct a medical image from medical imaging data. This further usage is described below also.

A general purpose large language model as used herein encompasses a large language model. The term `general purpose' is intended to emphasize or indicate that the large language model has not been specifically trained for medical applications or for the analysis of log data. This example may be beneficial because the information or data, which is in the semantically encoded database, is then directly available to the general purpose large language model.

General purpose large language models (LLMs) are designed to process and generate human-like text in response to general prompts. Unlike specialized models tailored for specific applications, these off-the-shelf LLMs offer broad utility, making them versatile for various industries and domains. Examples of such general-purpose LLMs include OpenAI's GPT series (e.g., GPT-4), Google's PaLM, Meta's LLaMA, and Anthropic's Claude. These models are pre-trained on extensive datasets and capable of non-specialized applications such as content generation, summarization, coding, and natural language understanding.

The inclusion of the semantically encoded database effectively modifies the general purpose large language model into a large language model that knows about and incorporates information about the protocol-specific lists. The semantic encoding enables the large language model to access the protocol-specific lists when constructing the response to prompts.

Semantic encoding is a process that converts text into numerical representations (vectors) that capture the meaning of the text rather than just its literal content. This is achieved using machine learning models, such as transformer-based neural networks, which analyze the context and relationships between words in a sentence. Each of the protocol-specific lists is mapped to a point in a high-dimensional space, where similar protocol-specific lists are placed closer together.

Semantically Encoded Databases use semantic encoding to index or reference the contents of the database. When incorporated into large language models (LLMs) using frameworks, such as Retrieval-Augmented Generation (RAG), these databases can retrieve contextually relevant entries. The RAG frameworks allow LLMs to query semantically encoded databases during inference to provide domain-specific knowledge. In examples, the semantic encoding enables the general purpose LLM to access the protocol-specific lists.

In another example, the method further comprises receiving medical imaging data descriptive of an internal anatomy of a subject. As used herein, medical imaging data represents raw data collected from a medical imaging system such as a tomographic medical imaging system. The medical imaging data may for example be computed tomography profiles, k-space data from a magnetic resonance imaging system, or data from other systems such as positron emission tomography systems or single-photon emission tomography systems.

The method further comprises receiving a selection of a chosen medical image generating protocol. This selection may, for example, be received automatically by a system or it may be selected by a user or operator. In some instances, the chosen medical imaging generating protocol might be typed into the general purpose large language model that has been modified by giving it access to the semantically encoded database. The method further comprises starting the task monitor system to generate control data by prompting the general purpose large language model of the task monitor system to perform the chosen medical image generating protocol. These prompts may take several forms. For example, an operator could click on a user interface object or select from a dropdown menu that automatically recalls a stored prompt that can be used to query the task monitor system.

In other examples, the user could write a free form sentence describing the imaging processing task to be performed, and this could be done for a variety of medical imaging generating protocols. For example, the construction of MRI Diffusion Tensor Imaging (DTI) images is very complex. The user could query the task monitor system with a query like: "Using the diffusion-weighted MRI data, generate a diffusion tensor image and visualize major white matter tracts in the brain. Provide a connectivity map and calculate key metrics such as fractional anisotropy and mean diffusivity for the selected tracts." The large language model would then query the semantically encoded database to search for a medical imaging generating protocol in the database that would enable this. Assuming that a corresponding protocol was available, the task monitoring system would then assist the operator to perform this task.

The system would be broadly applicable for various medical imaging modalities. For example, if it is in the semantically encoded database, the task monitor system could provide assistance in analyzing or generating CT images. An example prompt for performing CT angiography for vascular mapping might be: "From the contrast-enhanced CT angiography dataset, extract and segment the arterial tree of the abdomen. Identify and annotate key arteries such as the celiac trunk and superior mesenteric artery. Generate a 3D construction for surgical planning and measure the vessel diameters at specified locations." Prompts can be used to guide or monitor other medical image generating protocols and are described below.

As used herein, control data may encompass several different things. In one case the control data may represent commands that modify the behavior of the user interface, which is used to construct a medical image. In other cases, the control data may be text or commands which are output from the general purpose large language model.

The method further comprises constructing a medical image from the medical imaging data using at least one image processing application by using the control data to guide operation of the at least one image processing application. There are a variety of ways in which the control data could be used to guide the construction of the medical image. For example, the large language model may provide a detailed list or instructions which the operator of the user interface may follow. In other cases, the control data may provide commands which are used to modify the behavior of the user interface. For example, particular regions of the user interface may be modified or highlighted to guide the individual to perform the correct operations in the correct sequence.

Medical imaging data, as used here, encompasses the data acquired by a medical imaging system and which may be reconstructed into a medical imaging suitable for rendering on a display. A medical image, as used herein, encompasses an image which is constructed from medical imaging data and may be displayed to an individual. For example, it may be rendered using a monitor or other display. The medical image could encompass for example two, three, or four-dimensional images.

In another example, the method further comprises providing the medical image. Providing the medical image may for example encompass storing it in a database for further use. In other examples, providing the medical image may encompass rendering it on a display for a user or operator to view.

In another example, the method further comprises generating therapy control commands using the medical image. The therapy control commands may for example be commands which are used to control a therapy system such as a HIFU system, a gamma knife, a LINAC, or a radiotherapy system. The medical image in some examples may contain or emphasize anatomical data which is useful for generating the therapy control commands. A neural network or other algorithm may for example take the medical image as input and then output the therapy control commands.

In another example, the therapy control commands may be used for controlling a therapy system.

In another example, the method further comprises guiding the operation of the at least one image processing application before beginning of the chosen medical image generating protocol. In this example, the user for example may select to perform a particular or chosen medical image generating protocol. In this example this is done before the entire process is started. This example may be beneficial because the control commands may for example be useful in guiding the operator through the entire medical image generating protocol.

Various image generating protocols can be guided. For example, in MRI, a protocol might involve segmenting regions of interest such as brain structures or cardiac chambers using T1-weighted or T2-weighted imaging data, enabling precise identification of anatomical or pathological features. Another example is constructing diffusion tensor images (DTI) from raw diffusion-weighted MRI data to visualize neural pathways in the brain. In CT imaging, a protocol could involve segmenting lung nodules or lesions in thoracic scans to assist in early cancer detection or generating 3D volumetric constructions of vascular structures from contrast-enhanced CT angiography for preoperative planning. Further examples for spectral CT are given below. By monitoring the log file, the task monitoring system can monitor and assist the user in performing complex image processing tasks.

In another example, the method further comprises repeatedly retrieving the current log data by reading the log during performance of the chosen medical imaging protocol. The method further comprises repeatedly generating a list of performed actions by reformatting the current log data. The method further comprises inputting the list of performed actions into the general purpose large language model to update the task monitor system and update the control data. In this example, the system is used as a closed control loop. The method proceeds by repeatedly retrieving the current log data. This essentially provides an update as to what steps have been performed in the medical image generating protocol.

The list of performed actions then provides information on what the user has actually performed using the user interface. This up to date knowledge of what steps have been performed is then used to update the task monitor system and update the control data. This may be useful in several different situations. It for example may be useful if an operator is in the process of generating a medical image but then is uncertain how to proceed. The system can also be used to monitor if the operator is generating a medical image in an optimal or accepted manner. In both cases the updating of the task monitor system and updating the control data provides a way of guiding the user back on course for performing the chosen medical image generating protocol.

In another example, the method further comprises repeatedly querying the general purpose large language model of the updated task monitor system and checking if the list of performed actions is in accordance with the chosen medical image generating protocol and provide recommendations if it is not. In this example, the method goes through and monitors what the operator is doing and provides recommendations to correct if the operator is performing in a sub-optimal or non-standard fashion.

In another example, the method further comprises repeatedly querying the general purpose large language model of the updated task monitor system and updating the guidance of the at least one image processing application. This may, for example, be useful because the interval in which these queries are performed can be controlled and within this time period, the reactions of the system can essentially be real time.

Dual-energy computed tomography (CT), also known as spectral CT, utilizes at least two distinct X-ray energy spectra to generate detailed cross-sectional images of the body, enabling the differentiation of materials with varying attenuation properties at different energy levels. Spectral data obtained from this imaging technique can be processed to generate Spectral Base Images (SBI), which include advanced visualizations such as virtual non-contrast images, iodine concentration maps, and material decomposition images. This allows for enhanced diagnostic capabilities by extracting additional information from the imaging data. The described system leverages such data to construct semantically encoded databases from application usage logs related to protocols involving dual-energy CT and SBI generation, enabling efficient monitoring, analysis, and optimization of workflow performance in medical imaging environments.

In another example, the medical imaging data is spectral-based image series data for computed tomography. The medical image is a spectral computed tomography image. This example may be beneficial because the spectral-based image series data has information from several different energies of X-rays which is acquired for the computed tomography. This spectral information can be used to generate relatively complex spectral computed tomography images which may be constructed for special uses for diagnosis or for guiding other machines, such as a radiotherapy system. The fusion of a conventional CT image with spectral data may, in some examples, combine anatomical detail with material-specific information, such as iodine concentration or calcium distribution. This integration allows radiologists to assess structural and functional aspects of tissues in a single image, simplifying the correlation between anatomy and material composition. It may reduce the need for separate image comparisons and support more efficient clinical decision-making.

In another example, the spectral computed tomography image comprises a fused overlay of conventional computed tomography images with spectral data. This may, for example, provide for a specialized computer tomography image.

As a concrete example, a fused overlay in spectral CT may be realized using the following process: the process begins by selecting and loading the Spectral Base Image (SBI) series data. Afterward, users review the available viewing presets and screen layout options, choosing the Fusion Preset for visualization. For protocols such as urinary stones, users can select specific imaging options like conventional views, Uric Acid Removed [HU], Z Effective, or Uric Acid [HU]. The appropriate viewing option is then selected from the toolbar. Users can further choose from various data types for interpretation, including Conventional, MCI, kVpEquivalent, VirtualNonContrast, IodineNoWater, EffectiveZ, IodineMap, IodineNoCalciumHU, CalciumNoIodineHU, UricAcidNoCalciumHU, or CalciumNoUricAcidHU. These steps enable detailed, protocol-specific analysis and interpretation using fused overlays.

Examples may be used to guide this process: To utilize the Spectral Base Image (SBI) data, the system guides the user through a first step that involves opening the Spectral CT Viewer (SCTV) and loading the SBI series. Unlike standard DICOM files, SBIs can only be processed using SCTV or specialized spectral analysis software, as they may possibly be incompatible with generic DICOM viewers. Due to the larger file size and complexity of SBIs compared to conventional CT data, the system experiences a higher processing burden. This results in longer loading times and slower performance, especially for larger series, such as an SBI with 500 images, compared to a conventional CT study of the same size. The actual results and/or benefit may be dependent upon the actual field of view.

In a second step, users may be guided to review Viewing Presets and Screen Layout Options, possibly selecting a fusion image type preset for their specific clinical needs. While some layouts overlap across Clinical Preset areas, each preset may include unique configurations tailored to particular clinical applications. Users would then have the flexibility to save or delete custom Viewing Presets based on their preferences. A default viewing present may also be provided as a standard layout for initial review and analysis.

For a third step, the user may select an appropriate image processing protocol to suit the clinical application. For example, for urinary stone analysis, the protocol may include options such as Conventional CT, Uric Acid Removed [HU], Fused Conventional CT, Z Effective imaging, and Uric Acid [HU] in a "Slab" configuration. This may allow for the visualization and assessment of urinary stones by leveraging the benefits of both conventional and spectral imaging techniques to highlight material composition and relevant anatomical details.

In a fourth step the user may be guided to choose a viewing option of layout for the image.

In a fifth and final step, users may be guided to select one of the available data types to visualize and interpret the spectral CT images. The options in the urinary stone example may include Conventional, MCI, kVpEquivalent, VirtualNonContrast, IodineNoWater, EffectiveZ, IodineMap, IodineNoCalciumHU, CalciumNoIodineHU, UricAcidNoCalciumHU, and CalciumNoUricAcidHU. Each datatype may provide a specific perspective or material-specific analysis, enabling tailored visualization based on the clinical or diagnostic requirements of the case. This step may allow users to focus on the most relevant information for accurate interpretation.

In another example, the spectral computed tomography image comprises spectral CT data depicting different tomography spectra generated from multiple tube voltage keV values for a single region of interest. This may also be known as a "spectral magic glass image." Such images may provide a spectral fusion which shows visualization of different spectral view ports for different keV of two voltages for a particular region of interest. These images may, for example, be difficult for an operator to construct in a standard fashion. The control data may for example help to standardize the construction of these spectral magic glass images.

In an example of this, the system guides the user through the following steps to provide the spectral magic glass image: In a first step, the user is guided to select the Spectral Magic Glass icon from a menu to activate the tool. In a second step, the tool opens a Region of Interest (ROI) window on the main image. This window may include a movable box that displays multiple adjustable viewports of spectral results within the selected region. The display may dynamically update as the size of the Magic Glass window is changed, allowing focused visualization of the region. In step three, users may be guided to customize multiple viewports to display different datatypes for improved visualization of the spectral data, enabling detailed analysis tailored to specific diagnostic needs. This step-by-step approach enhances the user's ability to explore material-specific properties in a targeted and interactive manner. A user could for example be guided using a set of text instructions or shown prompts on the user interface.

In another example, the spectral computed tomography image is at least partially formed from photon-counted spectral CT data. In photon-counting computed tomography, individual X-ray photons and their energies are measured directly. In this technique, there is very detailed information about the spectral content of the X-rays available. It provides high resolution data that is particularly useful for analyzing smaller lesions or anatomical objects. This example may be beneficial because it may facilitate a more efficient workflow and more accurate image generation by possibly providing step-by-step guidance and optimizing parameter selection which could be difficult due to the large amount of spectral data available.

The at least one image processing application is automatically configured to generate the log during execution of the chosen medical image generating protocol. The generation of this log automatically means that the system may also be used to automatically monitor this and ultimately generate the control data which is used to guide usage of the user interface.

In another example, guiding the operation of the at least one image processing application comprises providing a text box listing subsequent user interface actions to follow. This, for example, could be provided directly by the general purpose large language model. This may provide a means of providing detailed instructions for the operator/user to follow.

In another example, guiding the operation of the at least one image processing application comprises providing graphical user interface prompts indicating the subsequent user interface actions to follow. The general purpose large language model could for example be used to generate commands directly which are used to control the graphical user interface. In other examples, the text output by the general purpose large language model could be parsed and used to look up graphical user interface prompts to perform using a lookup table or mapping.

In another example, the method further comprises providing user analytics by prompting the task monitor system with predetermined LLM prompts. These predetermined LLM prompts for performing analytics are an example of a prompt response that may be used to query the task monitor system. The response to the predetermined LLM prompts are however, not used to guide a user or monitor a current image processing task, but are instead used for providing analytics using information contained in the semantically encoded database.

For example, data from the log may be harvested from multiple medical imaging generating protocols and multiple image processing applications. This means that the semantically encoded database may contain a large number of protocol-specific lists for different protocols using different image processing applications. The data contained within this database may then be analyzed which may be used for performing tasks such as monitoring the general usage of the image processing applications for different image generating protocols as well as providing user statistics across various sites and locations as to what protocols they are performing and which applications. This for example may be used to assist in designing or modifying the image processing applications to perform better.

The use of the predetermined LLM prompts to query the general purpose large language model of the task monitor system may enable very complex analytics or statistics to be determined with minimal work. If one were interested in usage patterns or workflow insights of the at least one image generating protocol, LLM queries such as the following could be used:
"Summarize the most common sequences of actions performed by users during [specific medical image generating protocol]."
"Identify workflow bottlenecks or delays in [specific medical image generating protocol] ."
"What are the least-used features in [application name], and how does their usage vary across different protocols?"

Other types of analytics could include monitoring performance of the at least one image processing application by using LLM prompts such as these:
"Highlight any deviations from the standard protocol in the usage logs for [specific time frame]."
"Detect and list anomalies in the log data that suggest potential misuses or inefficiencies in [application name]."
"Generate a report summarizing the overall application usage, highlighting trends over [specific time period]."
"Provide insights into how the usage patterns differ between new users and experienced users of [application name]."
"Analyze the frequency of errors or repetitive actions and suggest improvements to the interface or workflow.

If one were interested in optimizing the at least one image processing application or how to provide training for users, the following LLM prompts may be useful:
"Based on the log data, suggest areas where additional training could improve user efficiency in [application name]."
"What features of [application name] are underutilized, and how might they be better integrated into workflows?"

In the above examples, the text "[application name]" could be replaced with the name or identifier of one or more applications to perform the query on.

As the above examples illustrate, the user analytics may provide detailed statistics about the usage of medical applications, such as those employed by radiologists during imaging workflows. By capturing and analyzing application usage data, the system can identify patterns in how radiologists interact with image processing software and protocols. This may enable the generation of detailed analytical insights, such as frequently used features, workflow bottlenecks, and common sequences of actions.

These insights can inform software developers and administrators about areas for optimization, improve training programs for radiologists, and enhance the overall usability of the applications. Additionally, monitoring usage patterns over time can help detect anomalies, such as deviations from standard protocols, which could indicate the need for quality assurance or additional training. This data-driven approach ensures that medical applications are continuously aligned with the needs of healthcare professionals, ultimately supporting more efficient and effective patient care.

In another example, the application usage data is retrieved using a log management application. The reformatting of the application usage data is preferably performed by querying the log management application with an SPL query. This may be beneficial because there are applications which are already existing and may be used for improving and processing the data from the log.

In one example, the log management application is a so-called Splunk tool. Usage of the Splunk tool may be particularly advantageous as it provides for an effective means of retrieving and then generating the protocol-specific lists. The Splunk tool can be employed for monitoring and summarizing log files generated during the use of the one or more medical imaging applications. Splunk has log analysis and data visualization capabilities that allow it to process large volumes of application usage data. By indexing the log files, Splunk can provide detailed summaries of user interactions, such as the frequency of specific actions, patterns in protocol execution, and potential anomalies in workflow usage. The integration of Splunk with other components of the system, such as the semantically encoded database and the general purpose large language model may ensure seamless.

In another example, the semantically encoded database is integrated into prompt responses using retrieval augmented generation. The use of retrieval augmented generation is beneficial because this provides for an effective means of integrating the data in the semantically encoded database into prompt responses of the general purpose large language model.

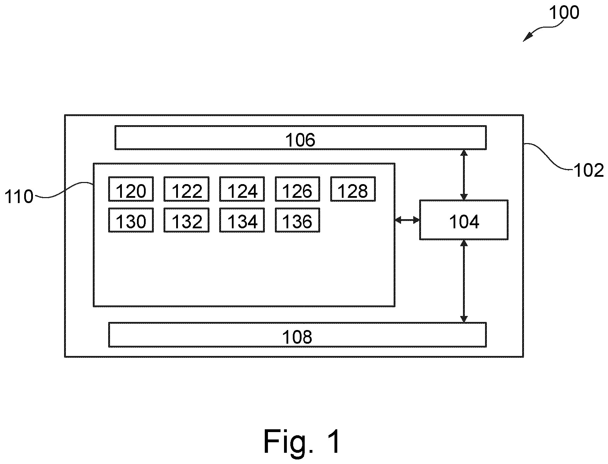

Fig. 1 illustrates an example of a medical system 100. The medical system 100 is shown as comprising a computer 102. The computer may represent one or more computers or computing systems at one or more locations. The computer 102 comprises a computational system 104. The computational system 104 may also represent one or more computational systems or computing cores located at one or more locations. The computational system 104 is shown as being in connection with an optional network interface or hardware interface 106. The network interface 106 may enable the computational system 104 to communicate with other computer systems as well as control other components of the medical system 100 if they are present.

The computational system 104 is also shown as being in communication with a memory 110 and an optional user interface 108. The user interface 108 may represent various types of interfaces of controls that enable an operator or controller to control the operation and function of the medical system 100. In some instances, the user interface 108 may also comprise a display or graphical user interface which enables the viewing of images.

The memory 110 is intended to represent various types of memory which are accessible to the computational system 104. For example, the memory 110 in some examples may be a non-transitory storage medium. The memory 110 is shown as storing machine-executable instructions 120. The machine-executable instructions 120 may enable the computational system 104 to control other components of the medical system 100 as well as perform various data analysis and image processing tasks. The memory 110 is further shown as containing a log 122 that records user actions performed during the use of an image processing application 124. The image processing application 124 may, for example, be used to perform reconstructions of medical images from medical imaging data as well as to perform various image processing and image manipulation tasks on existing medical images.

The image processing application 124 may, for example, be used to perform reconstructions of medical images from medical imaging data. For example, a medical image may be reconstructed from raw medical imaging data such as k-space data and/or CT projection data. The image processing application 124 may for example perform various image processing, constructing and image manipulation tasks on existing medical images. For example, an image may be constructed from medical imaging data to generate overlayed medical images and/or fused medical images.

Constructing a medical image may for example comprise reconstructing a medical image from raw medical image data such as k-space data and/or CT projection data. In other examples, constructing a medical image may for example comprise constructing a medical image from medical image data. Constructing a medical image also encompasses reformatting existing medical images or creating medical images derived from one or more existing medical images. Such constructing of a medical image may for example be to create overlayed medical images and/or fused medical images.

The memory 110 is further shown as containing application usage data 126 that was extracted from the log 122. For example, it may be a collection of actions that were performed using the image processing application 124. The log 122 could contain such things as details about which application caused the log entry as well as metadata such as the time or for what operation was performed. The memory 110 is further shown as containing protocol-specific lists 128 that represent individual medical image generating protocols performed by the image processing application 124. The protocol-specific lists 128 may be generated by formatting the application usage data 126 and dividing it into different protocols and reformatting so that it is human readable or readable by a large language model.

The memory 110 is further shown as containing a semantically encoded database 130 that is used to semantically encode different protocol-specific lists 128. The memory 110 is further shown as storing a general purpose large language model 132. The general purpose large language model 132 is used to provide a task monitor system 134 by configuring the general purpose large language model to integrate the semantically encoded database 130 into responses to prompts.

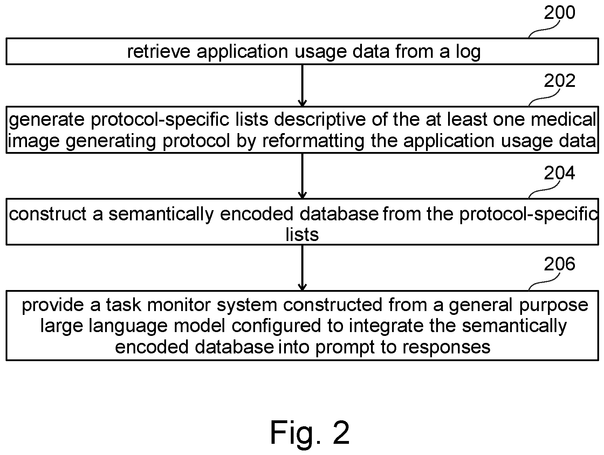

Fig. 2 shows a flowchart which illustrates a method of operating the medical system 100 of Fig. 1. In step 200 the application usage data 126 is received from the log 122. The application usage data is descriptive of user interface actions performed during at least one medical image generating protocol with the at least one image processing application 124. In step 202, the protocol-specific lists 128 are generated by reformatting the application usage data 126 such that the protocol-specific lists 128 are descriptive of the at least one medical image generating protocol. In step 204, a semantically encoded database 130 is constructed from the protocol-specific lists 128. In step 206, the task monitor system 134 is provided by constructing it from a general purpose large language model 132 that is configured to integrate the semantically encoded database 130 into prompt responses.

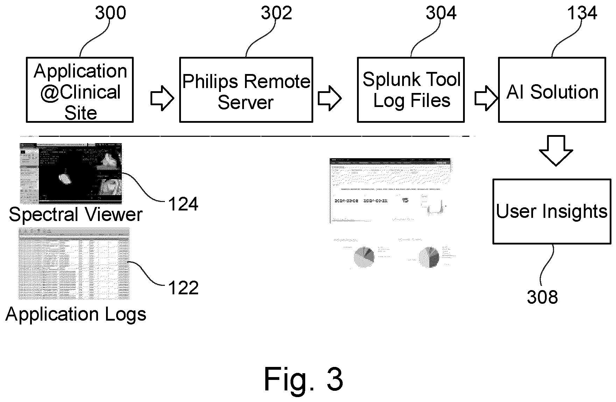

Fig. 3 illustrates one implementation of a method for performing an example. In this example, there is an application 300 run at a clinical site. This provides the image processing application 124 and the log 122 generated by the image processing application 124. The log data 122 may for example be passed to a remote server 302. This may for example be a server located at a client site such as a hospital or other healthcare facility or may even be provided in the cloud. In step 304, a log management application 304 is used to generate the protocol-specific lists 128. This may then be used by a task monitor system 134, which also may be referred to as an AI or artificial intelligence solution. As was mentioned above, in one example the task monitor system 134 comprises a general purpose large language model 132 that has had its behavior modified by having reference to a semantically encoded database 130 that contains a database of the protocol-specific lists 128. In addition to guiding the use of the image processing application 124, the task monitor system 134 may also be queried for details about the protocol-specific lists 128 in the semantically encoded database 130. This may be used to generate user insights 308, which may provide valuable statistics and information about the various clinical sites 300.

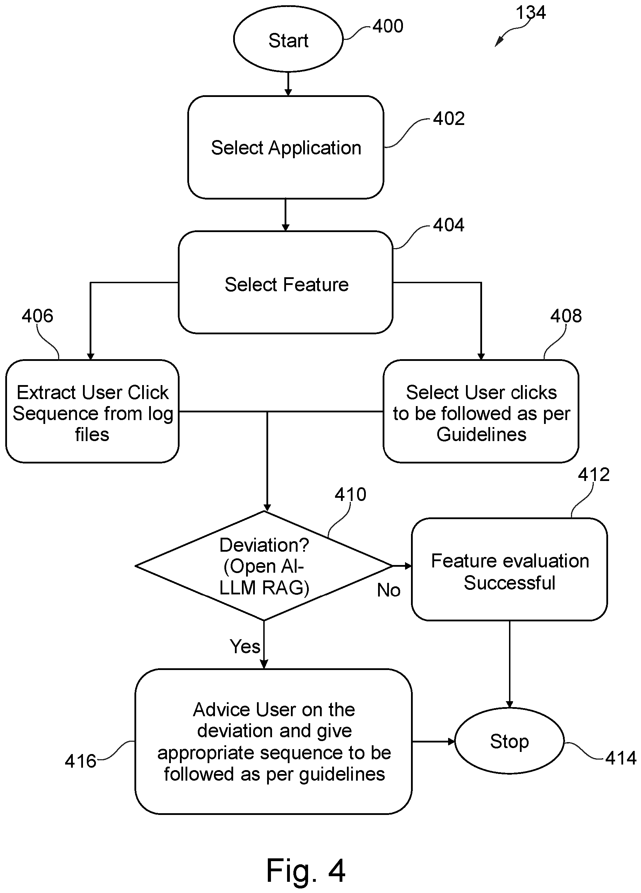

Fig. 4 illustrates one implementation of a task monitor system 134. Its functionality is represented by a flowchart. The method starts at start position 400. In step 402, a particular image processing application 124 is selected. After this, a particular feature 404 is selected. This may be equivalent to choosing a medical imaging protocol. After selection of this feature 404, two steps are performed in parallel. In step 406 the user click or application usage data 126 is extracted from the log 122. In step 408, which is performed in parallel to step 406, the application usage data 126, according to guidelines, is selected. In step 410, an artificial intelligence unit such as a large language model is used to compare the results in steps 406 and 408.

If there is no deviation between the two, 406, 408, the method proceeds to step 412 and the feature evaluation was successful. After step 412 is performed the method ends at step 414. In step 410, if there was a deviation the answer to the decision box 410 is yes and the method proceeds to step 416. In this step the user is advised on the deviation and given appropriate sequences to be followed as per guidelines. This may be providing updated details or instructions for the user to follow.

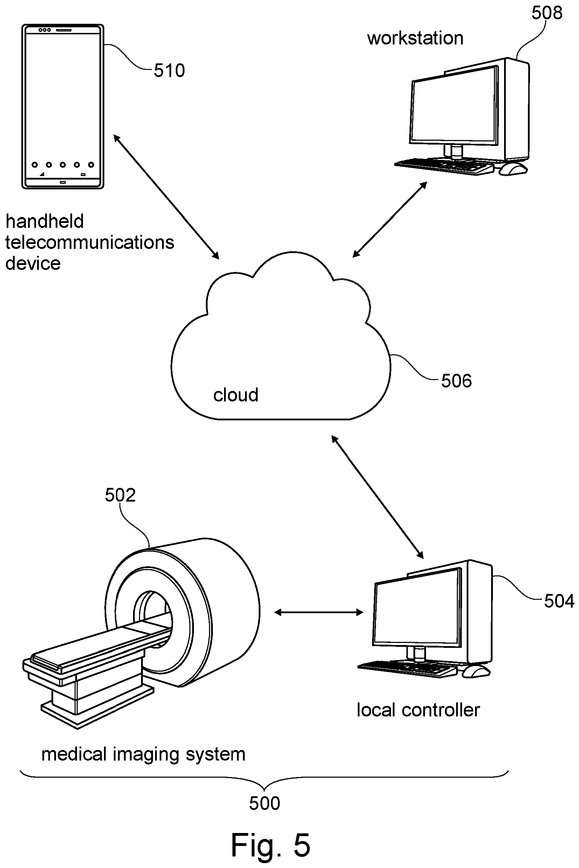

Fig. 5 illustrates a further example of a medical system 500 that could be used to implement examples. The medical system 500 comprises a medical imaging system 502 that could be used to acquire medical imaging data. The medical system 500 is further shown as comprising a local controller 504 for controlling and operating the medical imaging system 502 as well as a cloud-based computing system 502, a radiology workstation 504, and a handheld telecommunications device 506. The functionality of the computer 102 in Figs. 1 may be distributed amongst the various devices 504, 506, 508, and 510. The cloud-based computing system 506 could be made up of networked servers and/or virtual machines available over the internet or other network.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational systems to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the C programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, WLAN connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, television screen, touch screen, tactile electronic display, Braille screen, Cathode Ray Tube (CRT), storage tube, bi-stable display, electronic paper, vector display, flat panel display, Vacuum Fluorescent (VD) display, Light-Emitting Diode (LED) displays, Electro-Luminescent Display (ELD), Plasma Display Panels (PDP), Liquid Crystal Display (LCD), Organic Light-Emitting Diode (OLED) displays, a projector, and head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: optional network interface / hardware interface
- 108: user interface
- 110: memory
- 120: machine executable instructions
- 122: log
- 124: image processing application
- 126: application usage data
- 128: protocol-specific lists
- 130: semantically encoded database
- 132: general purpose large language model
- 134: task monitor system
- 136: prompt responses
- 200: retrieve application usage data from a log
- 202: generate protocol-specific lists descriptive of the at least one medical image generating protocol by reformatting the application usage data
- 204: construct a semantically encoded database from the protocol-specific lists
- 206: provide a task monitor system constructed from a general purpose large language model configured to integrate the semantically encoded database into prompt to responses
- 300: clinical site
- 302: remote server
- 304: log management application
- 306: optional AI Solution
- 308: optional user insights
- 400: start
- 402: select application
- 404: select feature
- 406: extract user click sequence from log files
- 408: select user clicks to be followed as per guidelines
- 410: Deviation (Open AI - LLM RAG)
- 412: feature evaluation successful
- 414: stop
- 416: advise user on the deviation and give appropriate sequence to be followed as per guideline
- 500: medical system
- 502: medical imaging system
- 504: local controller
- 506: cloud
- 508: workstation
- 510: handheld telecommunication device

## Claims

1. A medical method, wherein the method comprises:
- retrieving (200) application usage data (126) from a log (122), wherein the application usage data is descriptive of user interface actions performed during performing at least one medical image generating protocol with at least one image processing application (124);
- generating (202) protocol-specific lists (128) descriptive of the at least one medical image generating protocol by reformatting the application usage data;
- constructing (204) a semantically encoded database (130) from the protocol-specific lists; and
- providing (206) a task monitor system (134) constructed from a general purpose large language model (132) configured to integrate the semantically encoded database (130) into prompt responses.

2. The medical method of claim 1, wherein the method further comprises,
- receiving medical imaging data descriptive of an internal anatomy of a subject;
- receiving a selection of a chosen medical image generating protocol;
- starting the task monitor system to generate control data by prompting the general purpose large language model of the task monitor system to perform the chosen medical image generating protocol;
- constructing a medical image from the medical imaging data using the at least one image processing application by using the control data to guide operation of the at least one image processing application; and
- providing the medical image.

3. The medical method of claim 2, wherein the method further comprises generating therapy control commands using the medical image.

4. The medical method of claim 2 or 3, wherein the method further comprises guiding the operation of the at least one image processing application before beginning of the chosen medical image generating protocol.

5. The medical method of claim 2, 3, or 4, wherein the method further comprises:
- repeatedly retrieving current log data by reading the log during performance of the chosen medical image generating protocol;
- repeatedly generating a list of performed actions by reformatting the current log data; and
- inputting the list of performed actions into the general purpose large language model to update the task monitor system and update the control data.

6. The medical method of claim 5, wherein the method further comprises any one of the following:
- repeatedly querying the general purpose large language model of the updated task monitor system and checking if the list of performed actions is in accordance with the chosen medical image generating protocol and provide recommendations if it is not;
- repeatedly querying the general purpose large language model of the updated task monitor system and updating the guidance of the at least one image processing application; and
- combinations thereof.

7. The medical method of any one of claims 2 through 6, wherein the medical imaging data is spectral base image series data for computed tomography, and wherein the medical image is a spectral computed tomography image.

8. The medical method of claim 7, wherein any one of the following:
the spectral computed tomography image comprises a fused overlay of a conventional computed tomography image with spectral data;
the spectral computed tomography image comprises spectral CT data depicting different computed tomography spectra generated from multiple tube voltage KEV values for a single region of interest; and
the spectral computed tomography image is at least partially formed from photon-counted spectral CT data.

9. The medical method of any one of the preceding claims, wherein the at least one image processing application is automatically configured to generate the log during execution of the chosen medical image generating protocol.

10. The medical method of any one of the preceding claims, wherein guiding operation of the at least one image processing application comprises any one of the following:
- providing a text box listing subsequent user interface actions to follow;
- providing graphical user interface prompts indicating the subsequent user interface actions to follow; and
- combinations thereof.

11. The medical method of any one of the preceding claims, wherein the method further comprises providing user analytics (308) by prompting the task monitor system with predetermined LLM prompts.

12. The medical method of any one of the preceding claims, wherein the application usage data is retrieved using a log management application (304), and wherein reformatting the application usage data is preferably performed by querying the log management application with a SPL query.

13. The medical method of any one of the preceding claims, wherein the semantically encoded database is integrated into prompt responses using Retrieval Augmented Generation.

14. A computer program product comprising a computer-readable storage medium (110) having machine executable instructions embodied therewith, said machine executable instructions are configured to implement the method of any one of claims 1 through 13.

15. A medical system (100, 500) comprising:
- a memory (110) storing machine executable instructions (120);
- a computational system (104), wherein execution of the machine executable instructions causes the computational system to:
- retrieve (200) application usage data (126) from a log (122), wherein the application usage data is descriptive of user interface actions performed during performing at least one medical image generating protocol with at least one image processing application;
- generate (202) protocol-specific lists (128) descriptive of the at least one medical image generating protocol by reformatting the application usage data;
- construct (204) a semantically encoded database (130) from the protocol-specific lists; and
- provide (206) a task monitor system constructed from a general purpose large language model (132) configured to integrate the semantically encoded database into prompt responses.
